(19) Europäisches Patentamt
European Patent Office
Office européen des brevets

(11) Numéro de publication : **0 187 105 B1**

(12) # FASCICULE DE BREVET EUROPÉEN

(45) Date de publication du fascicule du brevet : **05.04.89**

(51) Int. Cl.⁴ : **C 07 D251/34**, C 08 G 18/02

(21) Numéro de dépôt : **85420184.5**

(22) Date de dépôt : **22.10.85**

(54) **Procédé de préparation de polyisocyanates polyisocyanurates par cyclotrimérisation catalytique de polyisocyanates.**

(30) Priorité : **30.10.84 FR 8416777**

(43) Date de publication de la demande : **09.07.86 Bulletin 86/28**

(45) Mention de la délivrance du brevet : **05.04.89 Bulletin 89/14**

(84) Etats contractants désignés : **AT BE CH DE GB IT LI LU NL SE**

(56) Documents cités :
**EP-A- 0 089 297**
**FR-A- 1 576 339**
**FR-A- 2 235 147**
**Le dossier contient des informations techniques présentées postérieurement au dépôt de la demande et ne figurant pas dans le présent fascicule.**

(73) Titulaire : **RHONE-POULENC CHIMIE**
**25, quai Paul Doumer**
**F-92408 Courbevoie Cédex (FR)**

(72) Inventeur : **Robin, Jean**
**21, rue Duguesclin**
**F-69006 Lyon (FR)**

(74) Mandataire : **Vignally, Noel et al**
**RHONE-POULENC INTERSERVICES Service Brevets Chimie Centre de Recherches de Saint-Fons B.P. 62**
**F-69192 Saint-Fons Cedex (FR)**

**EP 0 187 105 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne un perfectionnement à un procédé de préparation de polyisocyanates polyisocyanurates, par cyclotrimérisation catalytique partielle de polyisocyanates, cette réaction étant ultérieurement et volontairement arrêtée lorsque la teneur en trimère atteint la valeur désirée. Plus précisément, la présente invention concerne essentiellement un perfectionnement au procédé de désactivation du catalyseur, lors de l'emploi d'un catalyseur constitué par un composé à groupement aminosilylé.

Le brevet français n° 1 576 339 décrit un procédé d'obtention de solutions stables à la conservation de polymères à radicaux isocyanates, obtenus par polymérisation catalysée par un catalyseur basique, par addition auxdites solutions de composés organiques à hydrogène acide tels que les phénols, à raison d'au moins 1 mole de composé organique à hydrogène acide par équivalent de radicaux isocyanate libres.

La demande de brevet français n° FR-A-2 235 147 décrit un procédé de préparation de solution de polyisocyanates-isocyanurates exempts de diisocyanate libre par addition de 0,1 à 10 fois en moles d'alcools par rapport au diisocyanate libre.

Dans ces 2 documents de l'art antérieur, le but visé est de faire réagir l'isocyanate libre, qui se trouve dans le polyisocyanate, afin de le transformer ; le catalyseur de polymérisation ou de trimérisation n'est ni éliminé, ni transformé.

Dans le brevet français n° 8 203 799 publié sous le n° 2 522 667 et la demande de brevet européen n° 0 089 297 on a décrit un procédé de préparation de polyisocyanates polyisocyanurates par cyclotrimérisation catalytique de polyisocyanates, en utilisant à titre de catalyseurs des composés à groupements aminosilylés selon lequel lorsque la teneur désirée en groupement isocyanurate est atteinte, le catalyseur est détruit par addition d'un composé désactivateur, choisi parmi les composés organiques (A), porteurs au moins d'un groupement hydroxyle ou les composés résultant de la réaction de groupement isocyanate sur le composé organique (A), le composé organique (A), éventuellement porteur de groupement ou atomes inertes vis-à-vis des groupements isocyanates, étant choisi parmi les énols, les alcools, les phénols, les oximes ou les composés porteurs de groupement(s) hydroxysilylé(s).

L'addition du composé désactivateur est en général effectuée à une température comprise entre 50 et 180 °C et de préférence entre 80 et 130 °C, et en particulier à la température de cyclotrimérisation.

Il a été trouvé de manière surprenante que la désactivation des catalyseurs de cyclotrimérisation peut être avantageusement réalisée à une température inférieure à 50 °C dans le cas où on fait appel comme désactivateur à des composés organiques porteur d'au moins un groupe hydroxyle. On a même constaté que les polyisocyanates polyisocyanurates obtenus de cette façon présentent une stabilité encore améliorée par rapport à ceux obtenus selon le procédé du brevet français n° 8 203 799.

Cette stabilité améliorée se manifeste par l'inaptitude des polyisocyanurates obtenus par le procédé de la présente invention à générer du diisocyanate monomère pendant le stockage et même lors d'un chauffage de longue durée à une température supérieure ou égale à 50 °C. Il est particulièrement important d'éviter la présence dans les polyisocyanurates utilisés notamment pour l'obtention de revêtement de diisocyanates monomères dont la toxicité est bien connue.

Plus spécifiquement la présente invention a pour objet un procédé de préparation de polyisocyanates polyisocyanurates par cyclotrimérisation catalytique de polyisocyanates, en utilisant à titre de catalyseurs des composés à groupements aminosilysés puis, lorsque la teneur désirée en groupement isocyanurate est atteinte, destruction du catalyseur par addition d'un composé désactivateur choisi parmi les composés organiques porteurs d'au moins un groupement hydroxyle et éventuellement de groupements ou atomes inertes vis-à-vis des isocyanates, caractérisé en ce que l'addition du désactivateur est réalisée à une température inférieure à 50 °C.

La température à laquelle l'agent désactivateur peut être ajouté à la masse réactionnelle peut prendre toute valeur inférieure à 50 °C. Bien que cette température puisse être aussi basse que — 20 °C, il est d'un point de vue pratique sans intérêt de réaliser l'addition du désactivateur à une température inférieure à 10 °C. En règle générale la température d'addition du désactivateur est de préférence comprise entre 15 et 40 °C.

Les composés organiques comportant au moins un groupe hydroxyle qui conviennent comme désactivateurs pour la mise en œuvre du procédé selon l'invention sont ceux cités dans le brevet français n° 8 203 799 et la demande européenne n° 0 089 297. Plus spécifiquement on utilise les énols, les alcools primaires, secondaires ou tertiaires, les polyols primaires, secondaires ou tertiaires, les phénols, les polyphénols, les oximes, les composés à groupements hydroxysilylés tels que les silanols, les silanediols, les siloxanes ou les polysiloxanes à groupement(s) hydroxysilylé(s). Bien entendu, comme on l'a déjà dit, outre le groupement hydroxyle, le composé (A) peut éventuellement contenir tout groupement ou atome inerte, vis-à-vis des groupements isocyanates, tels que des groupements ester, éther, amide, des groupements organométalliques ou organo-métalloïdiques.

On mettra en œuvre, à titre d'énols des composés ayant au plus 10 atomes de carbone tels que les β-dicétones, les β-céto-esters et les β-cyano-esters. A titre illustratif on pourra citer l'acétylacétone, l'acétylacétate d'éthyle ou de méthyle ou de pentyle, le cyanoacétate d'éthyle.

On mettra en œuvre, à titre de mono-alcools, des « carbinols », primaires, secondaires ou tertiaires ayant, en général, de 1 à 8 atomes de carbone. Ces alcools peuvent éventuellement contenir des substituants inertes, vis-à-vis des groupements isocyanates tels que des groupements éther, ester, amide. Ces alcools peuvent ainsi être des hydroxyorganosilanes ou des hydroxyalkylsilanes. On reviendra dans la suite de l'exposé sur de tels composés.

Avantageusement, si on désire éliminer totalement le reliquat du désactivateur, on utilisera dans le cadre du procédé de la présente invention des mono alcools « simples » purement hydrocarbones et peu condensés en carbone (au plus 6 atomes de carbone), primaire ou secondaire tels que le méthanol, l'éthanol, le propanol, le n-butanol, l'isopropanol, le butanol secondaire. Préférentiellement, on utilisera des monoalcools primaires ou secondaires, ayant de 3 à 6 atomes de carbone, et dont la volatilité est de ce fait pas trop importante, tels que le butanol ou l'isopropanol.

On peut également utiliser, à titre de composé organique (A), un polyol éventuellement substitué par un ou plusieurs groupements inertes tels que précédemment définis. Dans un tel contexte, on peut citer :

— le glycérol,
— le propylèneglycol-1,3,
— le butanediol-1,4,
— le triéthylèneglycol,
— l'octanediol-1,3,
— le butynediol-1,4,
— le triméthylolpropane,
— l'éther monoéthylique ou méthylique du diéthylène glycol (diglyme).

Les polyols ont, en général, de 2 à 12 atomes de carbone et préférentiellement de 2 à 8 atomes de carbone.

Les phénols utilisables peuvent être des phénols mono ou polycycliques renferment éventuellement un ou plusieurs groupements phénoliques, et pouvant comporter divers substituants inertes vis-à-vis des groupements isocyanates, tels que des groupements alkyles, ester, éther, des atomes d'halogènes. A titre illustratif, on pourra citer parmi les phénols utilisables :

— le phénol,
— les crésols,
— les xylénols,
— le nonylphénol,
— les tertiobutylphénols,
— les dihydroxybenzène,
— le dihydroxy-4,4' biphényle,
— le dihydroxy-4,4' diphénylméthane,
— l'hydroxynaphtalène,
— le naphtalène diol.

Les oximes utilisables peuvent être des cétoximes ou des aldoximes obtenues par réaction d'hydroxylamine avec des aldéhydes ou des cétones linéaires ou cyclaniques ayant au plus 10 atomes de carbone, parmi les oximes on peut citer : l'acétone-oxime, la méthyléthylcétone-oxime, la cyclohexanone-oxime, l'hexanone-2 oxime, la cinnamaldéhyde oxime.

Parmi les composés à groupements hydroxysilylés, on pourra citer, à titre illustratif :

— le triméthylsilanol,
— le diméthylsilanediol,
— le triéthylsilanol,
— le diéthylsilanediol,
— le triphénylsilanol,
— le diphénylsilanediol,
— le dihydroxydiméthyldisiloxane,
— le dihydroxydiphényldisiloxane,
— le bis α,ω dihydroxy(octaphényltétrasiloxane).

On peut évidemment utiliser, dans le cadre de la présente invention, des composés organosiliciques porteurs de groupements hydroxyles non directement reliés à un atome de silicium. On pourra ainsi utiliser des hydroxyorganosilanes ou des hydroxyorganopolysiloxanes tels que :

— le triméthyl(hydroxyméthyl)silane,
— l'(hydroxybutyl)triméthylsilane,
— le bis(hydroxypropyl)diméthylsilane,
— l'hydroxyphényltriméthylsilane.

Les composés organosiliciques à groupements hydroxyles sont par exemple décrits dans l'ouvrage de Walter Noll « Chemistry and technology of silicones » — édition anglaise 1968.

A titre avantageux, on préfère utiliser dans le cadre de la présente invention un monoalcool primaire, secondaire ayant de 3 à 6 atomes de carbone. L'emploi de butanol ou d'isopropanol se révèle être particulièrement approprié.

La quantité d'agent désactivateur mise en œuvre peut être variable. Celle-ci n'est pas critique mais bien entendu elle dépendra de la quantité de catalyseur initialement introduit dans le polyisocyanate.

En général, la quantité d'agent désactivateur est telle, que le rapport molaire entre l'agent désactivateur et le catalyseur soit compris entre 0,5 et 2 et préférentiellement entre 0,8 et 1,5. Avantageusement, on utilise un rapport molaire voisin de 1.

Les composés aminosilylés utilisés comme catalyseurs de cyclotrimérisation dans le procédé selon l'invention sont ceux décrits et cités dans les demandes de brevet européen n° 0 057 653 et n° 0 089 297 c'est-à-dire les composés ayant pour formule (I) :

$$R_{(4-n)} — Si \left[ NR'R'' \right]_n$$

dans laquelle les divers symboles représentent respectivement :

— R : un radical monovalent de nature hydrocarbonée, aliphatique, cycloaliphatique, saturé ou insaturé, aryle, aralkyle ou alkylaryle, éventuellement substitué par des atomes d'halogène ou des groupements CN, deux radicaux R pouvant constituer ensemble un radical hydrocarboné divalent,

— R' : un radical monovalent choisi parmi les radicaux R, $SiR_3$, ou les radicaux amide de formule :

$$-CO-\underset{\displaystyle R}{\underset{|}{N}}-R'''$$

R''' représentant R ou $SiR_3$, R ayant la signification préalablement donnée, le radical R' pouvant éventuellement lorsqu'il ne représente pas un groupement amide ou un groupement $SiR_3$ constituer avec le radical R'' un radical hydrocarboné divalent,

— R'' : un radical monovalent ayant la même signification que le radical R ; ou un atome d'hydrogène lorsque R' n'est pas un radical amide,

— n : un nombre entier égal à 1 ou 2. Lorsque n est égal à 2, R' est un radical R.

Le catalyseur qui peut être un aminosilane, un diaminosilane, une silylurée ou un silazane est plus précisément représenté par la formule (I), dans laquelle les divers symboles représentent respectivement :

— R : un radical alkyle, alkényle ou halogénoalkyle ou halogénoalkényle ayant de 1 à 5 atomes de carbone et comportant de 1 à 6 atomes de chlore et/ou de fluor, des radicaux cycloalkyles, cycloalkényles et halogénocycloalkyles, halogénocycloalkényles ayant de 3 à 8 atomes de carbone et contenant de 1 à 4 atomes de chlore et/ou de fluor ; des radicaux aryles, alkylaryles et halogénoaryles ayant de 6 à 8 atomes de carbone et contenant de 1 à 4 atomes de chlore et/ou de fluor ; des radicaux cyanoalkyles ayant de 3 à 4 atomes de carbone ;

— deux symboles R portés par un même atome de silicium constituent entre eux un radical divalent ayant de 1 à 4 atomes de carbone.

— R' : un radical monovalent choisi parmi les radicaux R, $SiR_3$, et CO(NR)—R''', R''' représentant R ou $SiR_3$, R ayant la signification plus précise qui vient juste d'être donnée ci-dessus ; R' pouvant constituer avec R'' un radical alkylène ayant de 4 à 6 atomes de carbone.

— R'' : un radical alkyle ou alkényle ayant de 1 à 4 atomes de carbone, un radical cycloalkyle ou cycloalkényle ayant de 4 à 6 atomes de carbone nucléaire, un radical phényle ou tolyle ou xylyle, ou un atome d'hydrogène lorsque R' n'est pas un groupement amide.

Les composés aminosilylés, de formule (I), utilisés préférentiellement à titre de catalyseurs de cyclotrimérisation sont ceux dans la formule desquels les divers symboles représentent respectivement :

— R : un radical méthyle, éthyle, propyle, vinyle, phényle, ces radicaux pouvant éventuellement être chlorés et/ou fluorés,

— R' : un radical alkyle choisi parmi les radicaux méthyle, éthyle, propyle ou butyle,

un radical $SiR_3$, R ayant l'une des significations qui vient juste d'être définies, un radical carbonamide choisi parmi :

CO—NR—R

$$—CO—NR—SiR_3$$

R ayant l'une des significations qui vient juste d'être donnée

— R'' : un radical méthyle, éthyle, propyle ou butyle, ou un atome d'hydrogène.

Enfin R' et R'' peuvent ensemble constituer un radical butylène ou pentylène.

Comme on l'a déjà dit le catalyseur de cyclotrimérisation peut être un aminosilane, un diaminosilane, une monosilylurée, une disilylurée ou un silazane. Il est facile de déterminer la nature chimique exacte des divers composés à groupements aminosilylés utilisables, étant donné les diverses significations données précédemment aux divers radicaux R, R', R'', R'''. On notera en particulier que l'utilisation de silylurée obtenue par réaction d'amine secondaire et d'isocyanates-N silylé n'est pas envisagée. Ces silylurées sont impropres dans le procédé de cyclotrimérisation catalytique puisque libérant l'isocyanate silylé lors du chauffage.

Le composé à groupement aminosilylé sera un aminosilane lorsque n sera égal à l et lorsque R' représentera un radical R, les radicaux R et R'' ayant l'une des significations préalablement données, deux radicaux R pouvant constituer ensemble un radical divalent ou bien encore R' et R'' pouvant constituer ensemble un radical divalent. Parmi les aminosilanes on citera :

— le méthylamino triméthylsilane,
— le diméthylamino triméthylsilane,
— le diéthylamino triméthylsilane,
— le dibutylamino triméthylsilane,
— le diéthylamino diméthylvinylsilane,
— le diéthylamino diméthylphénylsilane.

Le composé à groupement aminosilylé sera un diaminosilane lorsque n sera égal à 2 et lorsque R' représentera le radical R, les radicaux R et R'' ayant l'une des significations préalablement données, deux radicaux R pouvant constituer ensemble un radical divalent ou bien encore R' et R'' pouvant constituer un radical divalent. Parmi les diaminosilanes on citera :

— le bis diméthylamino diméthylsilane,
— le bis dibutylamino diméthylsilane,
— le bis diméthylamino méthylphénylsilane.

Le composé à groupement aminosilylé sera une silylurée lorsque n sera égal à 1 et lorsque R' représentera le groupement carbonamide

$$-\underset{\underset{O}{|}}{C}-NRR'''$$

où R''' représente un radical R ou $SiR_3$, les radicaux R et R'' ayant l'une des significations préalablement données, 2 radicaux R pouvant constituer ensemble un radical divalent ou les deux radicaux R' et R'' (R' représentant alors R) pouvant constituer ensemble un radical divalent. Parmi les silylurées on citera :

— la N, méthyl N triméthylsilyl, N' méthyl N' butyl urée,
— le N triméthylsilyl N méthyl, N', N' diméthyl urée,
— la N triméthylsilyl N éthyl N', N' diméthyl urée,
— la N triméthylsilyl N butyl, N' butyl N' triméthylsilyl urée.

Le composé à groupement aminosilylé sera un silazane lorsque n sera égal à 1 et lorsque R' représentera un groupement $SiR_3$.

Les silazanes peuvent être symétriques ou dissymétriques ; on emploie préférentiellement les disilazanes symétriques, les deux groupements $SiR_3$ étant identiques.

Parmi les disilazanes utilisables on citera :

— l'hexaméthyldisilazane,
— l'heptaméthyldisilazane,
— le diéthyl-1,3 tétraméthyl-1,1,3,3 disilazane,
— le divinyl-1,3 tétraméthyl-1,1,3,3 disilazane,
— l'hexaméthyldisilazane,
— le diphényl-1,3 tétraméthyl-1,1,3,3 disilazane.

Enfin, on mentionnera tout particulièrement parmi les disilazanes l'hexaméthyldisilazane, l'heptamé-

thyldisilazane qui se révèlent être des catalyseurs tout particulièrement avantageux.

Dans le procédé de la présente invention, on peut cyclotrimériser en polyisocyanate polyisocyanurate tout polyisocyanate simple ou adduct, de nature aliphatique, cycloaliphatique ou aromatique et ceci à condition de bien choisir, en tant que catalyseur, le composé à groupement aminosilylé apte à cette réaction.

C'est ainsi que la cyclotrimérisation catalytique de polyisocyanates simples ou adducts, et dont les groupements isocyanates ne sont pas directement reliés à un noyau aromatique, peut être aisément effectuée en mettant en œuvre, en tant que catalyseur, un aminosilane, un diaminosilane, une silylurée ou un silazane, tels que précédemment définis.

Dans ce contexte, parmi les diisocyanates aliphatiques ou cycloaliphatiques, on citera :

— le tétraméthylène diisocyanate,
— le pentaméthylène diisocyanate,
— l'hexaméthylène diisocyanate,
— le diisocyanato-1,2 cyclohexane,
— le diisocyanato-1,4 cyclohexane,
— le bis(isocyanate-méthyl)-1,2 cyclobutane,
— le bis(isocyanato-4 cyclohexyl)-méthane,
— le triméthyl-3,3,5 isocyanatométhyl-5 isocyanato-1 cyclohexane.

Parmi ceux-ci on mentionnera tout particulièrement l'hexaméthylène diisocyanate.

Enfin, on peut citer, parmi les polyisocyanates adducts ou prépolymères utilisables à titre de polyisocyanates de nature aliphatique, les polyisocyanates modifiés qui sont obtenus en faisant réagir un excès de polyisocyanate aliphatique ou cycloaliphatique sur un composé comportant au moins deux groupements réactifs vis-à-vis des groupements isocyanates, tel qu'une diamine, un diacide. Les polyisocyanates modifiés qui peuvent être mélangés avec des polyisocyanates simples peuvent comporter des groupements urée, biuret, ester, siloxane.

On peut également, dans le cadre du procédé de la présente invention, cyclotrimériser en polyisocyanate polyisocyanurate tout polyisocyanate simple ou adduct de nature aromatique, c'est-à-dire ceux dans lesquels le groupement NCO est directement lié à un groupement aromatique. Pour y parvenir, on utilisera, en tant que catalyseurs à groupements amino-silylés, les aminosilanes, les diaminosilanes ou les silylurées telles que précédemment définies.

Parmi les diisocyanates aromatiques utilisables, on citera :

— le diisocyanato-1,4 benzène,
— les diisocyanato-toluène(-2,4 et -2,6 ou bien leurs mélanges),
— le diisocyanato-4,4' diphénylméthane,
— le diisocyanato-4,4' diphényléther,
— les polyméthylène, polyphénylène polyisocyanates.

On peut également utiliser à titre de polyisocyanate aromatique tout polyisocyanate adduct résultant de la polycondensation d'un excès de polyisocyanate sur un composé polyfonctionnel tel qu'une diamine, un diacide. Les polyisocyanates modifiés qui peuvent être mélangés avec des polyisocyanates simples peuvent comporter des groupements urée, biuret, ester, siloxane.

La quantité d'agent catalytique introduite dans l'isocyanate peut être variable, celle-ci exprimée pondéralement par rapport à l'isocyanate engagé étant habituellement comprise entre 0,1 et 10 % et de préférence entre 0,5 et 5 % ; on peut éventuellement introduire de petites quantités complémentaires de catalyseur durant la réaction.

Le procédé de cyclotrimérisation en polyisocyanate polyisocyanurate peut être effectué par simple chauffage des réactifs, à une température, en général, comprise entre 50 °C et 180 °C, de préférence entre 80 °C et 130 °C et habituellement autour de 100 °C.

Il est également possible, le cas échéant, d'effectuer la réaction de cyclotrimérisation en milieu solvant, ce dernier pouvant être un solvant peu polaire comme par exemple un hydrocarbure aliphatique ou aromatique, ou un ester ou un éther. On peut alors introduire le catalyseur dans le solvant et introduire cette solution dans l'isocyanate. On peut également introduire évidemment la solution catalytique dans l'isocyanate. Avantageusement, le procédé est effectué sans solvant.

Lorsque la teneur en isocyanurate atteint la valeur désirée, on abaisse la température de la masse réactionnelle à une valeur inférieure à 50 °C et ajoute le désactivateur dont la nature et la proportion ont été précédemment définis.

On peut alors éventuellement éliminer le polyisocyanate monomère excédentaire par tout moyen connu et parvenir à un polyisocyanate polyisocyanurate renfermant une teneur excessivement réduite en isocyanate monomère, ainsi qu'une faible teneur en isocyanate dimère.

Les polyisocyanates polyisocyanurates tels que ceux issus d'hexaméthylène diisocyanate sont des composés bien connus et particulièrement intéressants comme constituants de base pour vernis et peinture.

Les exemples qui suivent illustrent l'invention.

Exemple 1

Dans un ballon de 3 litres muni d'un agitateur et d'un thermomètre, on charge 2 400 g de diisocyanato-1,6 hexane. On chauffe au bain d'huile jusqu'à 95 °C et on ajoute alors 48 g d'hexaméthyldisilazane ; on maintient à 100° pendant 2 h 15 mn. A ce moment-là on dose une teneur en groupes NCO de 0,990/100 g. La masse réactionnelle est alors fractionnée en trois parties égales A, B et C.

Partie A : on maintient la température à 100° et ajoute 8 g de n-butanol puis on laisse refroidir. Une petite quantité (10 à 20 g) est maintenue 20 h à 100°, pour vérifier que la trimérisation est bien arrêtée ; au bout de ce temps, la teneur en groupements NCO et la viscosité n'ont pas changé par rapport aux valeurs mesurées au moment du blocage.

Le reste de A est ensuite évaporé au moyen d'un évaporateur couche mince à film agité, jusqu'à obtention de trimère ne renfermant plus de diisocyanate libre (teneur inférieure ou égale à 0,1 % en poids). On obtient ainsi le trimère A'.

Partie B : on la refroidit rapidement à 30° et ajoute alors 8 g de n-butanol. Comme précédemment, on vérifie sur une petite quantité que la trimérisation est bien arrêtée ; après 20 h à 100°, la teneur en groupe NCO est restée constante. Le reste de la fraction B est alors évaporé comme pour A et donne le trimère B' renfermant moins de 0,1 % en poids de diisocyanate libre.

Partie C : on la refroidit rapidement à 5° et y ajoute alors 8 g de n-butanol. On opère comme pour B :

— on constate sur un échantillon de cette partie C que l'arrêt de la trimérisation est effectif (teneur en NCO inchangée après 20 n à 100°).

— on évapore le reste et obtient le trimère C' renfermant au plus 0,1 % de diisocyanate libre.

Les fractions A', B' et C' sont stockées à température ordinaire. On mesure leur stabilité en prélevant un échantillon que l'on maintient 60 jours à 60° et dont on détermine ensuite la teneur en diisocyanate libre. On constate que, pour A', cette teneur atteint 0,6 % en poids, alors qu'elle est restée pratiquement inchangée pour B' et C' (inférieure à 0,2 % en poids).

Exemple 2

On recommence la trimérisation du diisocyanatohexane comme décrit dans l'exemple 1.

Lorsque la teneur en groupes NCO est de 0,975 pour 100 g, on refroidit rapidement à 30°. La masse réactionnelle est fractionnée en 5 parties égales : D, E, F, G, H.

Dans chacune de ces fractions, on ajoute 0,06 mole de :

| | |
|---|---|
| — isopropanol | (fraction D) |
| — méthyl-2 propanol | (fraction E) |
| — triméthylsilanol | (fraction F) |
| — phénol | (fraction G) |
| — éthylèneglycol | (fraction H) |

Une petite quantité de chacune de ces fractions est maintenue 20 h à 100° : on constate que le blocage obtenu est bien effectif car la teneur en NCO n'a pratiquement pas varié. Ces cinq fractions sont évaporées, comme pour l'exemple 1, jusqu'à obtention des trimères correspondants D', E', F', G' et H', dont la teneur en diisocyanate libre est inférieure ou égale à 0,1 % en poids.

Après conservation à 60° pendant 60 jours, la teneur pondérale en diisocyanate libre est restée pratiquement inchangée (inférieure à 0,2 % en poids).

## Revendications

1. Procédé de préparation de polyisocyanates polyisocyanurates par cyclotrimérisation catalytique de polyisocyanates, en utilisant à titre de catalyseurs des composés à groupements aminosilysés puis, lorsque la teneur désirée en groupement isocyanurate est atteinte, destruction du catalyseur par addition d'un composé désactivateur choisi parmi les composés organiques porteurs d'au moins un groupement hydroxyle et éventuellement de groupements ou atomes inertes vis-à-vis des isocyanates, caractérisé en ce que l'addition du désactivateur est réalisée à une température inférieure à 50 °C.

2. Procédé selon la revendication 1, dans lequel le composé désactivateur est choisi parmi les énols, les alcools primaires, secondaires ou tertiaires, les polyols primaires, secondaires ou tertiaires, les phénols ou les polyphénols, les cétoximes, les composés organosiliciques à groupement(s) hydroxysilylé(s).

3. Procédé selon l'une des revendications 1 ou 2, dans lequel le composé désactivateur est choisi parmi les monoalcools primaires, secondaires ou tertiaires ayant de 1 à 8 atomes de carbone.

4. Procédé selon l'une des revendications 1 à 3, dans lequel le composé désactivateur est un monoalcool primaire ou secondaire ayant de 3 à 6 atomes de carbone.

5. Procédé selon l'une quelconque des revendications 1 à 4, caractérisé en ce que la quantité de désactivateur exprimée en mole par mole de catalyseur est comprise entre 0,5 et 2.

## Claims

1. Process for preparing polyisocyanurate-polyisocyanates by catalytic cyclotrimerization of polyisocyanates, using by way of catalysts compounds containing aminosilyl groups, and then, when the desired content of isocyanurate groups is reached, destruction of the catalyst by adding a deactivating compound chosen from organic compounds bearing at least one hydroxyl group and optionally groups or atoms which are inert towards isocyanates, characterized in that the addition of the deactivator is carried out at a temperature below 50 °C.

2. Process according to claim 1, wherein the deactivating compound is chosen from enols, primary, secondary or tertiary alcohols, primary, secondary or tertiary polyols, phenols or polyphenols, ketoximes and organosilicon compounds having hydroxysilyl group(s).

3. Process according to one of claims 1 or 2, wherein the deactivating compound is chosen from primary, secondary or tertiary monohydric alcohols having from 1 to 8 carbon atoms.

4. Process according to one of claims 1 to 3, wherein the deactivating compound is a primary or secondary monohydric alcohol having from 3 to 6 carbon atoms.

5. Process according to any one of claims 1 to 4, characterized in that the amount of deactivator expressed as mole per mole of catalyst is between 0.5 and 2.

## Patentansprüche

1. Verfahren zur Herstellung von Polyisocyanaten-Polyisocyanuraten durch katalytische Cyclotrimerisation von Polyisocyanaten, wobei als Katalysatoren Verbindungen mit aminosilylierten Gruppen verwendet werden, dann, wenn der gewünschte Gehalt an Isocyanuratgruppen erreicht ist, Zerstörung des Katalysators durch Zugabe einer desaktivierenden Verbindungen, ausgewählt unter den organischen Verbindungen, die mindestens eine Hydroxylgruppe und eventuell Gruppen oder Atome, die gegenüber den Isocyanaten inert sind, tragen, dadurch gekennzeichnet, daß die Zugabe des Desaktivators bei einer Temperatur unterhalb 50 °C stattfindet.

2. Verfahren gemäß Anspruch 1, dadurch gekennzeichnet, daß die desaktivierende Verbindung ausgewählt wird unter den Enolen, den primären, sekundären oder tertiären Alkohlen, den primären, sekundären oder tertiären Polyolen, den Phenolen oder Polyphenolen, den Ketoximen, den Organosiliciumverbindungen mit hydroxysilylierter(en) Gruppe(n).

3. Verfahren gemäß einem der Ansprüche 1 oder 2, dadurch gekennzeichnet, daß die desaktivierende Verbindung ausgewählt ist unter den primären, sekundären oder tertiären Monoalkoholen mit 1 bis 8 Kohlenstoffatomen.

4. Verfahren gemäß einem der Ansprüche 1 bis 3, dadurch gekennzeichnet, daß die desaktivierende Verbindung ein primärer oder sekundärer Monoalkohol mit 3 bis 6 Kohlenstoffatomen ist.

5. Verfahren gemäß einem der Ansprüche 1 bis 4, dadurch gekennzeichnet, daß die Menge Desaktivator, ausgedrückt in Mol pro Mol Katalysator, zwischen 0,5 und 2 liegt.